# EUROPÄISCHE PATENTSCHRIFT

(11) **EP 0 867 198 B1**
(45) Veröffentlichungstag und Bekanntmachung des Hinweises auf die Patenterteilung: **30.07.2003**
(21) Anmeldenummer: 98810215.8
(22) Anmeldetag: 13.03.1998
(51) Int. Cl.: A61M 39/02

(54) **Konnektierungssystem für medizinische Anwendungen**
Coupling system for medical applications
Système de couplage pour applications médicales

(30) Priorität: 26.03.1997 CH 73097
(43) Veröffentlichungstag der Anmeldung: 30.09.1998
(73) Patentinhaber: Disetronic Licensing AG, 3401 Burgdorf (CH)
(72) Erfinder: Schäfer, Dieter, 65428 Rüsselsheim (DE); Frei, Thomas, 3432 Lützelflüh (CH)

(56) Entgegenhaltungen:
- US-A- 4 634 424
- US-A- 4 781 693
- US-A- 4 995 866
- US-A- 5 573 517
- US-A- 5 607 443

## Beschreibung

Die vorliegende Erfindung bezieht sich auf ein Konnektierungssystem für medizinische Anwendungen gemäss dem Oberbegriff des Patentanspruchs 1.

Diverse Konnektierungssysteme zwischen einem Katheter und einem Körper sind in der Medizinaltechnik bekannt. Aus der Patentschrift WO 89/06987 ist ein Konnektierungssystem zwischen einem im menschlichen Körpergewebe implantierbaren Portsystem und einem Katheter bekannt. Im menschlichen oder tierischen Körper wird eine Portkammer angeordnet, an der ein im Inneren des Körpers verbleibender Katheter und ein von aussen in die Portkammer zu stossender äusserer Katheter befestigt werden können. Im Inneren der Portkammer befindet sich eine Silikonmembran, welche vom äusseren Katheter durchstossen werden kann. Um die Membran beim Durchstossen möglichst nicht zu verletzen, ist am äusseren Katheter eine Kanüle angebracht, wobei das die Silikonmembran durchstossende Ende der Kanüle mit einem kugelförmigen Abschluss versehen wird. Ein solches System wird unter dem Warenzeichen PERCUSEAL® vertrieben. Nachteilig an diesem System ist, dass der auf die Kanüle aufgespritzte kugelförmige Abschluss aus Kunststoff gefertigt wird. Die Oberfläche des Kunststoffes ist mit Brauen versehen und sehr rauh, was beim Einführen der Kanüle in die Portkammer zu Verletzungen der Silikonmembran führt. Eine verletzte Silikonmembran kann ihre Funktion, die Verhinderung von Fremdkörpereintritten in den Körper, nicht mehr im geforderten Mass erfüllen.

Im weiteren sind Konnektierungssysteme zwischen einem Kanülenträger für subkutane oder intravenöse Stahl- oder Teflonkanülen und einem Katheter bekannt. Häufig ist dabei die von einem auf der Haut befestigten Träger gehaltene Stahl- oder Teflonkanüle mit einer Einstichhilfe lösbar verbunden und wird mit deren Hilfe unter die Haut oder in eine Vene gestossen. Sitzt die Kanüle richtig, wird die Einstichhilfe entfernt und an deren Stelle wird ein Infusionskatheter mit dem Kanülenträger gekoppelt. Die Koppelung erfolgt mittels einer am Infusionskatheter befestigten spitzen Koppelungskanüle, welche in eine entsprechende Aufnahme im Kanülenträger gestossen wird. Das Einführen der spitzen Koppelungskanüle ist häufig problematisch, weil ein falscher Einführungswinkel dazu führen kann, dass die Spitze in die Seitenwand eindringt, anstatt im geführten Kanal zu verlaufen. Der Infusionskatheter muss zudem mittels einer Befestigungsvorrichtung am Kanülenträger befestigt werden, damit sich die Koppelungskanüle nicht allzu leicht vom Kanülenträger lösen kann. Ein solcher Koppelungsmechanismus ist zum Beispiel aus der Patentschrift WO 94/20160 bekannt.

Als nächstliegender Stand der Technik gilt die US-A-4 634 424). Die hierin geschützte Erfindung offenbart ein Konnektierungssystem mit einem implantierbaren Septum und ein implantierbares Reservoir mit einem Medikament, welches (im menschlichen Körper) mehrfach nachgefüllt werden kann. Zur Nachfüllung des Reservoirs werden zwei Nadel verschiedener Dicke gebraucht.

Ebenfalls bekannt sind Konnektierungssysteme zwischen Arzneimittelampullen und einem Infusionskatheter. Meist ist dabei der Infusionskatheter am der Ampulle zugewandten Ende mit einem weiblichen Luer versehen, während die Ampulle selbst mit einem männlichen Luer versehen ist. Zur Konnektierung wird der weibliche Luer über den männlichen Luer gestossen. Nachteilig an diesem System ist, dass die Verbindung zwischen männlichem und weiblichen Luer sehr toleranzabhängig ist und demnach bereits leichte Winkelabweichungen zu einem Leck führen können.

Hier will die Erfindung Abhilfe schaffen. Der Erfindung liegt die Aufgabe zugrunde, ein Konnektierungssystem zur Konnektierung eines Arzneimittel enthaltenden Behälters und eines Arzneimittel aufzunehmenden Behälters oder Körpers anzugeben, welches sowohl mehrmals verwendet werden kann, wie auch gewisse Herstellungstoleranzen duldet, ohne dass dies Einfluss auf Dichtigkeit des Systems hat.

Die Erfindung löst die Aufgabe mit einem Konnektierungssystem, welches die Merkmale des Anspruchs 1 aufweist.

Die durch die Erfindung erreichten Vorteile sind im wesentlichen darin zu sehen, dass Dank der erfindungsgemässen Materialwahl und -verarbeitung die elastische Membran in einer entsprechenden Kammer nicht mehr in dem Mass verletzt wird, wie bei Verwendung einer aufgespritzten Kunststoffkugel auf eine Kanüle und zudem durch die Elastizität der Membran gewisse Toleranzen in der Herstellung ausgeglichen werden können.

Bevorzugte Ausführungsbeispiele der Erfindung sind in den Figuren dargestellt.

Es zeigen:
- Fig. 1: ein erfindungsgemässes Konnektierungssystem
- Fig. 2: ein erfindungsgemässes Konnektierungssystem mit einem über die Kanüle gestossenen kugelförmigem Abschluss
- Fig. 3: ein erfindungsgemässes Konnektierungssystem mit einem auf die Kanüle aufgesetzten kugelförmigem Abschluss
- Fig. 4: ein Konnektierungssystem mit einem gestauchten Kanülenabschluss
- Fig. 5: ein erfindungsgemässes Konnektierungssystem für ein Portsystem
- Fig. 6: ein erfindungsgemässes Konnektierungssystem für eine subkutane oder intravenöse Teflon- oder Stahlkanüle
- Fig. 7: ein erfindungsgemässes Konnektierungssystem für eine Arzneimittelampulle

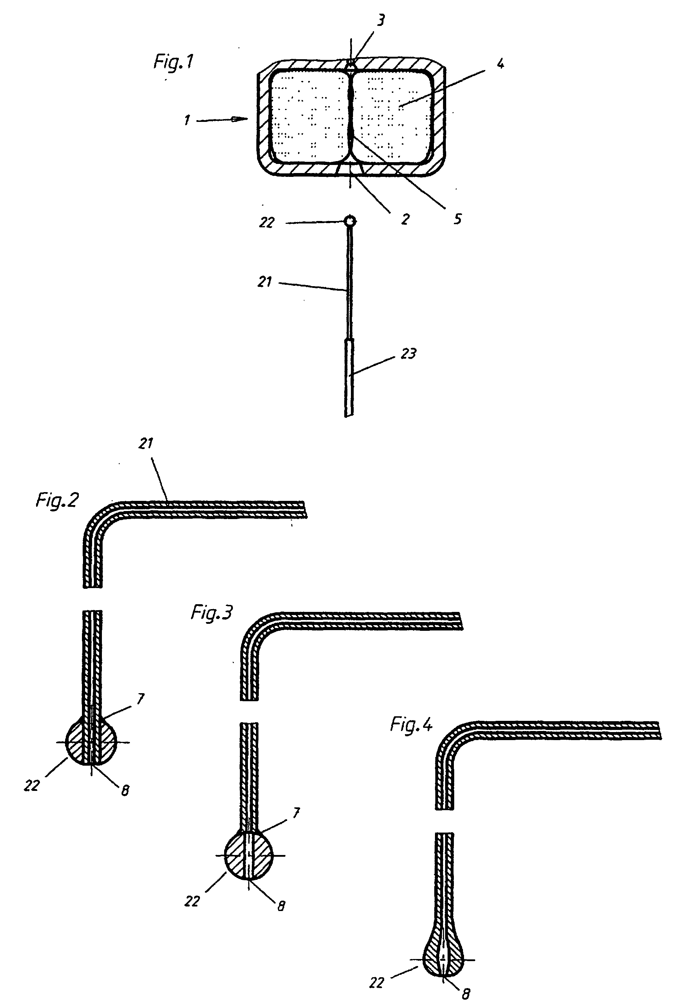

Wie in Figur 1 dargestellt, besteht ein erfindungsgemässes Konnektierungssystem aus einer Membrankammer 1, mit zwei gegenüberliegenden, unterschiedlich grossen Öffnungen 2,3 , einer zwischen den Öffnungen 2,3 angeordneten, aus einem elastischen Material bestehenden Membran 4, welche im Zentrum einen Verbindungskanal 5 zwischen den beiden Öffnungen 2,3 der Kammer 1 aufweist. Eine Kanüle 21 mit kugelförmigen Abschluss 22 kann durch die ihr zugewandte Öffnung 2 der Kammer 1 durch den Kanal 5 zur gegenüberliegenden Öffnung 3 gestossen und auf gleichem Weg wieder entfernt werden. Die der Kanüle 21 gegenüberliegende Öffnung 3 ist im Durchmesser kleiner als der kugelförmige Abschluss 22 der Kanüle 1.
Die Membran 4 in der Kammer 1 steht unter mechanischem Druck, so dass sich der Kanal 5 von selbst schliesst und damit das Eindringen von Fremdkörpern in und durch den Kanal 5 verhindert. Beim Einführen der Kanüle 21 muss der kugelförmige Abschluss 22 den Kanal 5 öffnen. Die Kanüle wird jederzeit von der Membran 4 umgeben, so dass wenn der kugelförmige Abschluss 22 der Kanüle 21 die gegenüberliegende Öffnung 3 erreicht, die Abdichtung gewährleistet ist.

Die Ausgestaltung der Kammer 1 und der Membran 4 ist derart, dass der kugelförmige Abschluss 22 der Kanüle 21 nach dem Einführen in der gegenüberliegenden Öffnung 3 gehalten wird. Da der Durchmesser der gegenüberliegenden Öffnung 3 kleiner ist als der Durchmesser des kugelförmigen Abschlusses 22 der Kanüle 21, kann die Kanüle nicht gänzlich durch die Kammer 1 gestossen werden, sondern nur bis zum Anschlag des kugelförmigen Kanülenabschlusses 22 an der Kammerinnenwand auf der Höhe der gegenüberliegenden Öffnung 3. Die Membran 4 ist im Bereich der gegenüberliegenden Öffnung 3 derart geformt, dass der kugelförmige Kanülenabschluss 22 an die Kammerinnenwand der gegenüberliegenden Öffnung 3 gepresst wird.

Um die Membran 4 möglichst nicht zu verletzen wird der kugelförmige Kanülenabschluss 22 vorzugsweise aus einem anorganischen Material gefertigt und wird mit einer fein bearbeiteten Oberfläche versehen, deren Rauhwerte maximal 0,8 µm betragen. Wie aus den Figuren 2-4 ersichtlich, kann der kugelförmige Abschluss, welcher mit einer diametralen, durchgehenden Bohrung 8 versehen ist, entweder über die Kanüle 21 geschoben werden (Fig. 2) oder an die Kanüle 21 angesetzt werden (Fig. 3). Ebenfalls denkbar ist, dass die Kanüle selbst aus einem Material gefertigt ist, welches durch Druck auf den Kanülenabschluss, selbst abgerundet wird. In diesem Fall ist ein kugelförmiger Abschluss eher unwahrscheinlich, weshalb Figur 4 entsprechend ausgestaltet wurde.
Bevorzugtes Material für den kugelförmigen 22 oder abgerundeten 22 Abschluss der Kanüle 21 ist Chrom-Nickelstahl.
Bevorzugtes Material für die Membran 4 ist Silikon.

Beim Auf- oder Ansetzen eines kugelförmigen Abschlusses 22 an die Kanüle 21, wird die Befestigung mittels einer Lötnaht 7 erfolgen.

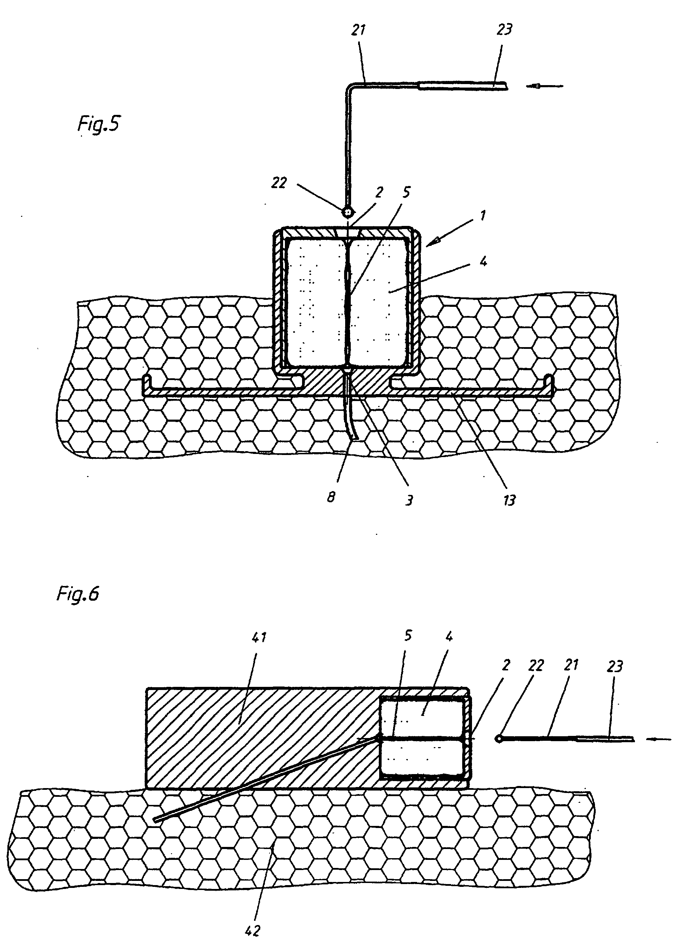

Das erfindungsgemässe Konnektierungssystem kennt verschiedene Anwendungsarten. In Figur 5 ist ein Portsystem dargestellt, welches in den Körper eines Organismus' eingesetzt wird. Bei diesen Portsystemen ist unmittelbar mit der Membrankammer 1 ein Verankerungsteller 13 verbunden, welcher die Membrankammer 1 im Gewebe verankert.

Die Kanüle 21 wird durch die ihr zugewandte Öffnung 2 und anschliessend durch den Membrankanal 5 bis zur ihr abgewandten Öffnung 3 geschoben. Befindet sich der kugelförmige Kanülenabschluss 22 bei der ihm abgewandten Öffnung 3, umschliesst die Membran 4 den kugelförmigen Kanülenabschluss 22 derart, dass die Kanüle 21 an die abgewandte Öffnung 3 gedrängt wird. Unmittelbar an die abgewandte Öffnung 3 schliesst ein innerer Katheter 8 an, so dass ein Arzneimittelkanal von einem Infusionsschlauch 23 bis in den Organismus besteht.

Anstelle der Verbindung mit einem Infusionsschlauch 23, kann die Kanüle 21 mit kugelförmigem Abschluss 22 auch an einem Injektionsgerät angeordnet sein, so dass mittels des Injektionsgerätes eine Injektion unmittelbar durch die Kammer 1 in einen hinter der Kammer liegenden Körper erfolgt.

Fig. 6 zeigt ein erfindungsgemässes Konnektierungssystem zwischen einem Kanülenträger 41 für eine subkutane Teflonkanüle 42 und einem Infusionsschlauch 23.

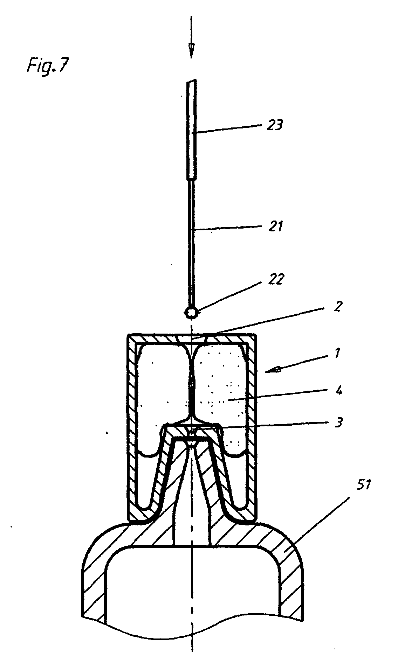

Fig. 7 zeigt ein erfindungsgemässes Konnektierungssystem zwischen einer Ampulle 51 und einem Infusionsschlauch 23. Die Kammer 1 wird an einer Ampulle 51 angebracht. Über das Konnektierungssystem kann Arzneimittel in die Ampulle 51 abgegeben werden oder aber die Ampulle geleert werden.

## Patentansprüche

1. Konnektierungssystem zur Konnektierung eines ein Arzneimittel enthaltenden Behälters und eines ein Arzneimittel aufzunehmenden Behälters oder Körpers, umfassend eine Kanüle (21) mit abgerundetem Kanülenabschluss (22) und eine eine elastische Masse (4) beinhaltende Kammer (1) mit zwei Öffnungen (2,3), wobei die elastische Masse (4) die beiden Öffnungen (2,3) durch einen Kanal (5) verbindet und wobei der abgerundete Kanülenabschluss (22) aus einem organischen Material besteht, **dadurch gekennzeichnet, dass** der Kanülenabschluss (22) kugelförmig ist.

2. Konnektierungssystem nach Anspruch 1, **dadurch gekennzeichnet, dass** die Rauhwerte des Kanülenabschlusses (22) maximal 8 Mikrometer betragen

3. Konnektierungssystem nach Anspruch 2, **dadurch gekennzeichnet, dass** der Kanülenabschluss (22) aus Metall, vorzugsweise aus Chrom-Nickelstahl gefertigt ist.

4. Konnektierungssystem nach einem der Ansprüche 1-3 **dadurch gekennzeichnet, dass** der Kanal (5) durch die elastische Membran (4) selbstschliessend ist.

5. Konnektierungssystem nach einem der Ansprüche 1-4, **dadurch gekennzeichnet, dass** die Kammer (1) zugleich eine Portkammer ist.

6. Konnektierungssystem nach einem der Ansprüche 1-4, **dadurch gekennzeichnet, dass** die Kammer 1 Verbindungsstück zu einem Kanülenträger (41) ist.

7. Konnektierungssystem nach einem der Ansprüche 1-4, **dadurch gekennzeichnet, dass** die Kammer (1) Verbindungsstück zu einer Arzneimittelampulle (51) ist.

8. Konnektierungssystem nach einem der Ansprüche 1-4, **dadurch gekennzeichnet, dass** die Kammer (1) Teil einer Arzneimittelampulle (51) ist.

## Claims

1. A connecting system for connecting a container containing a drug and a container or body to receive a drug, including a cannula (21) with a rounded-off cannula termination (22) and a chamber (1) having two openings (2, 3), the chamber containing an elastic material (4), wherein the elastic material (4) connects the two openings (2, 3) through a passage (5) and wherein the rounded-off cannula termination (22) comprises an organic material, **characterised in that** the cannula termination (22) is spherical.

2. A connecting system according to claim 1 **characterised in that** the roughness values of the cannula termination (22) are at a maximum 8 micrometres.

3. A connecting system according to claim 2 **characterised in that** the cannula termination (22) is made from metal, preferably chromium-nickel steel.

4. A connecting system according to one of claims 1 to 3 **characterised in that** the passage (5) is self-closing by the elastic diaphragm (4).

5. A connecting system according to one of claims 1 to 4 **characterised in that** the chamber (1) is at the same time a port chamber.

6. A connecting system according to one of claims 1 to 4 **characterised in that** the chamber (1) is a connecting portion to a cannula carrier (41).

7. A connecting system according to one of claims 1 to 4 **characterised in that** the chamber (1) is a connecting portion to a drug ampoule (51).

8. A connecting system according to one of claims 1 to 4 **characterised in that** the chamber (1) is part of a drug ampoule (51).

## Revendications

1. Système de connexion pour connecter un récipient contenant un médicament et un récipient ou un corps recevant un médicament, comprenant une canule (21) avec fermeture de canule (22) arrondie et une chambre (1) avec deux ouvertures (2, 3), contenant une masse élastique (4), la masse élastique (4) reliant les deux ouvertures (2, 3) par un canal (5) et la fermeture de canule arrondie (22) étant en un matériau organique, **caractérisé en ce que** la fermeture de canule (22) est en forme de bille.

2. Système de connexion. selon la revendication 1, **caractérisé en ce que** les valeurs de rugosité de la fermeture de canule (22) sont au maximum de 8 micromètres.

3. Système de connexion selon la revendication 2, **caractérisé en ce que** la fermeture de canule (22) est réalisée en métal, de préférence en acier au nickel-chrome.

4. Système de connexion selon l'une des revendications 1 à 3, **caractérisé en ce que** le canal (5) se ferme automatiquement par la membrane élastique (4).

5. Système de connexion selon l'une des revendications 1 à 4, **caractérisé en ce que** la chambre (1) est en même temps une chambre d'accès.

6. Système de connexion selon l'une des revendications 1 à 4, **caractérisé en ce que** la chambre (1) sert de pièce de liaison avec un support de canule (41).

7. Système de connexion selon l'une des revendications 1 à 4, **caractérisé en ce que** la chambre (1) sert de pièce de liaison avec une ampoule de médicament (51).

8. Système de connexion selon l'une des revendications 1 à 4, **caractérisé en ce que** la chambre (1) fait partie d'une ampoule de médicament (51).
